(19) Europäisches Patentamt
European Patent Office
Office européen des brevets

(11) Publication number: 0 095 512 B1

(12) EUROPEAN PATENT SPECIFICATION

(45) Date of publication of patent specification: 04.02.87

(51) Int. Cl.⁴: A 61 K 31/16

(21) Application number: 82104654.7

(22) Date of filing: 27.05.82

# (54) Antipsoriatic composition.

(43) Date of publication of application:
07.12.83 Bulletin 83/49

(45) Publication of the grant of the patent:
04.02.87 Bulletin 87/06

(84) Designated Contracting States:
AT BE CH DE FR GB IT LI LU NL SE

(56) References cited:
ARZNEIMITTEL FORSCHUNG, vol. 15, no. 7, July 1965, J. MOLNAR: "Die pharmakologischen Wirkungen des Capsaicins, des scharf schmeckenden Wirkstoffes im Paprika", pages 718-727

CHEMICAL ABSTRACTS, vol. 72, no. 11, March 16, 1970, page 228, abstract no. 53317b, Columbus, Ohio, US, J. SMITH et al.: "Effects of capsaicin on human skin, liver and epidermal lysosomes"

CHEMICAL ABSTRACTS, vol. 52, no. 3, February 10, 1958, columns 2334i/2335a, Columbus, Ohio, US, K.E. SCHULTe et al.: "Influence of the ointment base on the activity of capsaicin-containing ointments"

(73) Proprietor: Bernstein, Joel E.
615 Brierhill Road
Deerfield Illinois 60015 (US)

(72) Inventor: Bernstein, Joel E.
615 Brierhill Road
Deerfield Illinois 60015 (US)

(74) Representative: Baillie, Iain Cameron et al
c/o Ladas & Parry Isartorplatz 5
D-8000 München 2 (DE)

EP 0 095 512 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an antipsoriatic composition.

Psoriasis is a common chronic skin condition for which exist today a limited number of modestly effective agents, these being primarily topical corticosteroids and coal tar preparations. Various topical steroids effectively used to treat psoriasis of the skin includes fluocinolone acetonide, fluorandrenolide, and triamcinolone acetonide are usually applied as creams or ointments. These topical steroids are most effective if covered with a polyethylene film which preferably is sealed with tape. Thin polyethylene gloves are used for treating the hands and fingers. Treatment of psoriatic skin can also include daily removal of the scales by applying soap and water and scraping gently with a soft brush, followed by the application of a keratolytic ointment.

I have observed psoriasis seems to be much less common in Mexicans and Orientals than in American Caucasians and blacks. Mexicans and Orientals eat substantially more spicy food containing red pepper than either Caucasians or Blacks. Capsaicin (the active principle in red pepper that makes the red pepper hot) has been found to be an effective treatment for psoriasis of the skin when applied topcially in divided doses. Exposure of the treated psoriatic skin to small doses of ultraviolet light also assists treatment.

The present invention provides the use of capsaicin with a pharmaceutically acceptable carrier of a lotion, cream or ointment, the capsaicin being present in an amount not less than 0.01 percent by weight of the carrier, for the manufacture of a medicament for the treatment of psoriasis.

In the practice of the present invention, capsaicin is distributed according to known techniques in various pharmaceutically acceptable carriers such as edmulsions, solutions, suspensions including lotions, creams and ointments. Some of these carriers contain volatile diluents such as alcohol and glycol and also may contain wetting agents, emulsifying and suspending agents.

Capsaicin the active ingredient in the psoriasis preparation is a pungent principle in fruit of the various species of Capsicum or Solanaceae (pepper plants). Chemically, Capsaicin is known as trans-8-methyl-N-vanillyl-6-nonenamide or (E)-N-[(4-hydroxy-3-methoxyphenyl)-methyl)]-8-methyl-6-nonenamide. It structure is:

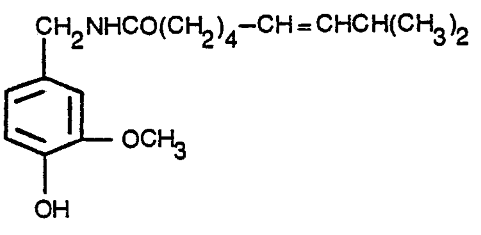

Capsaicin, commercially available from the Sigma Chemical Company, is present in the pharmaceutically acceptable carrier in an amount of not less than 0.01 percent by weight and is preferably present in the range of from 0.01 percent by weight to 1 percent by weight.

If capsaicin is present in the pharmaceutically acceptable carrier in an amount less than 0.01 percent by weight, then there is insufficient concentration of the capsaicin to provide effective therapy. If the capsaicin is present in an amount greater than about 1 percent by weight of the pharmaceutically acceptable carrier, then the reaction of the psoriatic skin to the topical application is too painful. I have found the intial treatment of psoriatic skin with capsaicin results in an intense red painful reaction but the psoriatic skin becomes quite tolerant to capsaicin applications upon subsequent treatment.

After treatment of patients with capsaicin in a pharmaceutically acceptable carrier, exposure to small amounts of ultraviolet light in the range of between 3 to 5 MED per exposure in some cases hastens clearing and produces a better therapeutic benefit than the use of capsaicin alone. *MED stands for "minimum erythermal dose", see the Handbook of Nonprescription Drugs, Sixth Edition, American Pharmaceutical Association, 1979.* The capsaicin is preferably administered topically in divided doses 2 to 4 times a day with partial clearing of the psoriasis being observable in a five to ten day range.

The following examples further illustrate the present invention:

Example 1

An ointment containing 0.01% by weight capsaicin was applied twice daily to the abdomen by a 28 year old white patient with extensive psoriasis involving most of the body. A plain emollient ointment was applied to the other areas of the skin. Within seven days of treatment the abdomen was nearly clear of psoriatic lesions, while the rest of the body was unchanged.

Example 2

A 0.05% by weight capsaicin solution was applied to psoriatic elbow lesions of a 30 year old white patient with mild psoriasis limited to the elbows. The solution was applied 2 to 3 times daily. The patient was first observed again two weeks later and the elbows were completely clear of psoriatic lesions.

Example 3

A cream having 0.1% by weight capsaicin was applied 3 times a day by a 25 year old white patient with psoriasis affecting primarily the extensor surfaces of the arms and legs. Within 7 days of application redness had decreased dramatically, scaling was reduced and the lesions had decreased significantly in size.

A 1% by weight solution of capsaicin was prepared in an aqueous/alcohol vehicle and applied 4 times daily to the arms, legs, chest and back of a 58 year old black patient with psoriasis. Almost complete resolution of the psoriatic lesions was observed after 5 days of such treatment.

Example 5

A cream containing 0.1% by weight capsaicin was applied 4 times daily to the arms of a 49 year old white patient with psoriasis over the extensor surfaces of both arms. One arm was exposed to ultraviolet light for from 2 to 5 minutes in increasing doses every day for 8 days using a hot quartz lamp as the source of the ultraviolet light. After 8 days both arms were significantly improved. However, the arm exposed to ultraviolt light was completely clear of psoriatic lesions, while the other arm still had some small lesions.

Example 6

A 0.5% by weight capsaicin ointment was applied twice daily by a 42 year old black patient with psoriasis involving the arms, legs, back, abdomen, and buttock. After 1 week the patient showed significant clearing of the lesions and at this time exposure to small (2—3 minutes) of ultraviolet light was initiated daily with resulting total clearing noted by the end of the following week.

**Claims**

1. The use of capsaicin with a pharmaceutically acceptable carrier of a lotion, cream or ointment, the capsaicin being present in an amount not less than 0.01 percent by weight of the carrier, for the manufacture of a medicament for the treatment of psoriasis.

2. The use according to claim 1, wherein said capsaicin is present in the range of from 0.01 to 1 percent by weight of said carrier.

**Patentansprüche**

1. Die Verwendung von Capsaicin mit einer pharmazeutisch verwendbaren Trägersubstanz in Form einer Flüssigkeit, Creme oder Salbe, wobei das Capsaicin in einer Menge von mindestens 0,01 Gew.% der Trägersubstanz vorhanden ist, zum Erzeugen eines Medikaments zur Behandlung der Psoriasis.

2. Erfindung nach Anspruch 1, dadurch gekennzeichnet, daß das Capsaicin in einer Menge von etwa 0,01 bis etwa 1 Gew.% der Trägersubstanz vorhanden ist.

**Revendications**

1. Utilisation de la capsaïine avec un support pharmaceutiquement acceptable pour une lotion, une crème ou un onguent, la capsaïcine étant présente en une quantité au moins égale à environ 0,01% en poids du support, pour la fabrication d'un médicament pour le traitement du psoriasis.

2. Utilisation selon la revendication 1, dans laquelle ladite capsaïcine est présente à raison d'environ 0,01 à 1% en poids dudit support.